# EUROPEAN PATENT SPECIFICATION

(11) **EP 0 684 985 B1**
(45) Date of publication and mention of the grant of the patent: **02.07.1997**
(21) Application number: 94906280.6
(22) Date of filing: 09.02.1994
(51) Int. Cl.: C12M 1/24

(54) **VENTING DEVICE**
ENTLÜFTUNGSVORRICHTUNG
DISPOSITIF DE DECOMPRESSION

(30) Priority: 17.02.1993 EP 93301172
(43) Date of publication of application: 06.12.1995
(73) Proprietor: UNIPATH LIMITED, Basingstoke, Hampshire RG24 OPW (GB)
(72) Inventor: CARR, Anthony Hugh, Stevington, Bedford MK43 7QB (GB)
(74) Representative: Matthews, Heather Clare
(86) International application number: GB9400255
(87) International publication number: WO9419451

(56) References cited:
- EP-A- 0 126 390
- FR-A- 2 029 242
- US-A- 4 066 067
- US-A- 4 416 984
- US-A- 5 051 360

## Description

### Field of the invention

This invention relates to a venting device, and in particular to a venting device for a culture bottle having a seal incorporating a septum.

### Background to the invention

When cultures are grown in a microbiological laboratory to test specimens, exampled by blood cultures, it can happen that, due to organism growth, substantial pressure develops inside the culture bottle. Sometimes, positive pressure can build up to the point where danger is involved and, in this case, the only alternative to disposing of the bottle, with consequent loss of the specimen, is to vent the bottle. In the latter case, however, it is necessary to ensure that venting does not lead to erroneous results due to the introduction of extraneous organisms into the bottle by the venting process.

### Summary of the invention

According to the present invention, in its broadest aspect, there is provided a venting device for a culture bottle having a septum, comprising a needle carrier having a venting volume in communication with a needle, and a membrane-carrying structure within which the needle carrier and needle are movably sealed, the arrangement being such that the device is operable, when positioned with the membrane between the needle and the septum, firstly to move the membrane away from its position between the needle and the septum and secondly to move the needle to pierce the septum in order to vent excess volume into the venting volume.

The membrane-carrying structure preferably comprises an outer sleeve closed at one end by a frangible membrane, and a guide tube axially slidable through the outer sleeve, for cutting and deflecting the membrane out of the path of the needle carrier, the device having an unused rest condition in which the forward end of the guide tube is retracted behind the membrane and the forward end of the needle is retracted behind the forward end of the guide tube, an intermediate operational condition in which the guide tube has been moved forwardly to cut and deflect the membrane out of the path of the needle, and a final operational condition in which the needle carrier has been moved forwardly through the guide tube, in use to enable the needle to penetrate the septum of the culture bottle and vent the bottle to the interior of the needle carrier behind the needle.

The above-defined venting device thus provides a means of venting whereby, assuming the needle to be aseptic within the device prior to use, this asepticity is substantially maintained right up to the moment of penetration of the bottle septum. Avoidance of contact of the needle with the membrane, e.g. a metallic foil, closing the outer sleeve of the device is most important to this end.

Preferably, therefore, the interior of the needle carrier behind the needle contains a microbiologically effective filter for assisting the prevention of movements of organisms into or out of the bottle.

In order to maintain the integrity of the culture bottle, the needle carrier is preferably releasable out of the forward end of the guide tube, whereby in use the needle remains in place extending through the septum of the culture bottle. The venting device is thus preferably in the form of a sterile package, the outer sleeve and guide tube being disposable after use and the needle carrier and needle being disposable with the culture bottle when microbiological testing has been completed. The package may be rendered aseptic by an irradiation process.

Features of the culture bottle are important in relation to use of the above-described venting device.

According to another aspect of the invention, therefore, there is provided, in combination with the venting device, an overcap for the culture bottle which has an entry throat in which the needle carrier is accommodated when the needle is left extending through the septum of the culture bottle. The entry throat of the overcap preferably carries an axial guide for a part of the needle carrier of reduced cross section in which the needle is mounted.

According to still another aspect of the invention, there is provided culture bottle examination apparatus which includes means for detecting pressure variations in the bottle, a venting device as heretofore defined, and means for operating the above-defined venting device if an excess bottle pressure is detected.

Conveniently, said examination apparatus may comprise a multi-bottle carrier for a plurality of culture bottles, means for repeatedly scanning the bottles to detect pressure variations in the bottles, and means for moving a venting device into an operating position at any bottle at which an excess pressure is detected. In this apparatus, it is preferred if the scanning mechanism for a pressure detecting means is also employed to move a venting mechanism incorporating the venting device to a bottle requiring venting.

The complete venting mechanism also may incorporate a motorised drive for operating the venting device in the manner hitherto described, and also a magazine or carrier for replacing a spent venting device with a fresh one.

An example of apparatus for monitoring the growth of micro-organisms in culture bottles, comprising examination apparatus as aforesaid, is claimed and described in Patent Application No. PCT/GB92/01327 (WO 93/03178).

An embodiment of venting device in accordance with the invention is now exemplified in the following description, making reference to the accompanying drawings, in which:-
Figures 1 to 6 show a preferred embodiment of venting device in six successive stages of operation;
Figure 7 shows a culture bottle monitoring apparatus in which the venting device may be incorporated; and
Figure 8 shows the manner in which a venting device may be incorporated in the apparatus of Figure 7.

### Detailed Description of the Drawings

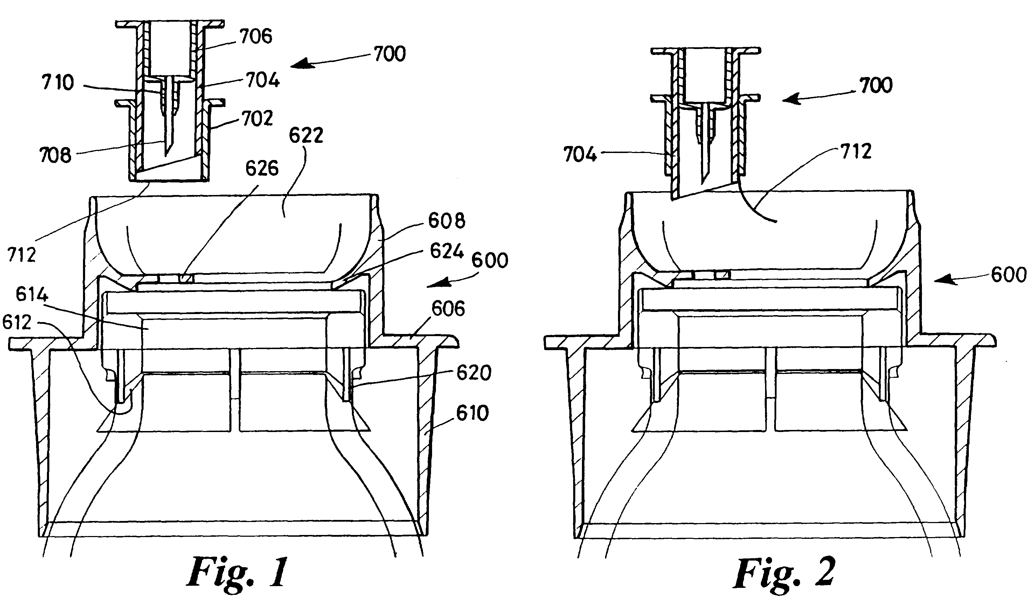

Referring first to Figures 1 to 6, there is shown the top of a culture bottle fitted with a bottle seal incorporating a septum and with an overcap 600 from which a tamper-evident tear-off portion has been removed. This overcap is claimed and described in more detail in European Patent Application No. 94906281.4 (WO 94/19452).

The illustrated overcap 600 has all round a generally square lateral projection 606 which is relatively thin, so that the body of the cap has cylindrical sections 608 and 610 respectively above and below the square projection. The lower cylindrical section 610 has internally a lip 612 which snap-fits over the bottle seal 614.

The upper surface of the projection 606 is on one side marked with a barcode for bottle identification purposes, and on the opposite side, adjacent one corner, has a port at which, when the bottle is in use in an incubation carrier, an LED uniquely associated with each particular bottle cap can be viewed. Thus, an LED may operate if excess pressure develops in the bottle. Safe and easy handling, even by an operator wearing gloves, is facilitated by the square lateral projection 606, and the LED port imparts a tactile and visible asymmetry which facilitates replacement of the bottle in the correct orientation, e.g. for reading of the barcode, which uniquely identifies the bottle independently of its position in the carrier.

Internally, as shown in the drawings, the overcap 600 has an inner dependent sleeve 620 within the lower outer cylindrical section 610. The lip 612 which snap-fits over the bottle seal 614 is provided at the bottom of this inner sleeve 620.

Above the inner sleeve, the upper cylindrical section 608 is formed internally with a relatively deep throat 622, which at the bottom is terminated by a curved, inwardly and downwardly directed deformable lip 624 which engages the top of the bottle seal, taking up manufacturing tolerances. The deep throat 622, in conjunction with the square lateral projection 606, reduces risk of "needle-stick" injuries when a hypodermic syringe is used, for example to sample the bottle contents. Also adjacent the bottom of the throat 622, the cap 600 is formed internally with an apertured tab 626 which constitutes a guide for the needle of a bottle venting device in accordance with the present invention.

The venting device, generally referenced 700, is used to vent the culture bottle if an excess and possibly dangerous pressure develops in the bottle during use, e.g. incubation in an incubation carrier.

The venting device comprises an outer sleeve 702, a guide tube 704 slidable in the outer sleeve, a needle carrier 706 slidable in the guide tube, and a short needle 708 mounted in a forward (bottom) end portion 710 of the needle carrier which is of reduced cross-section. In the rest condition of the device, shown in Figure 1, the forward (bottom) end of the outer sleeve 702 is sealed closed by a membrane such as a metallic foil 712.

The entire venting device constitutes a small disposable package, entirely moulded of plastics material except for the needle. The package, and in particular the interior thereof, is rendered sterile by irradiation. Moreover, behind the needle, the interior of the needle carrier 706 accommodates a microbiologically effective filter element (not shown) to contain aerosols and prevent movement of micro-organisms either into or out of the culture bottle during venting.

The venting device is shown in various stages of operation in Figures 1 to 6. Figure 1, as stated, shows the normal rest condition. Sterility is maintained inside, especially of the needle 708, by the sealed closure 712.

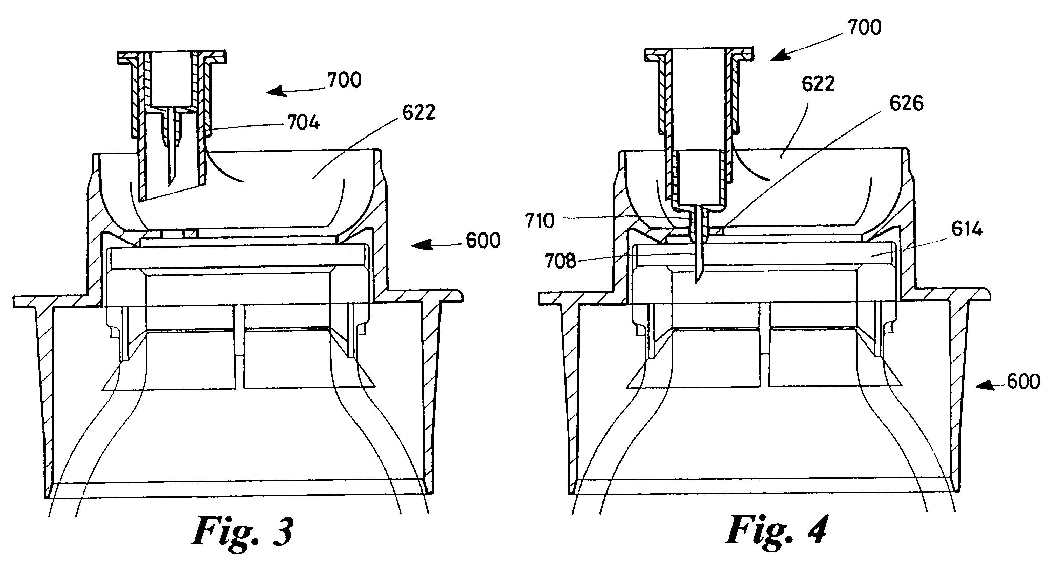

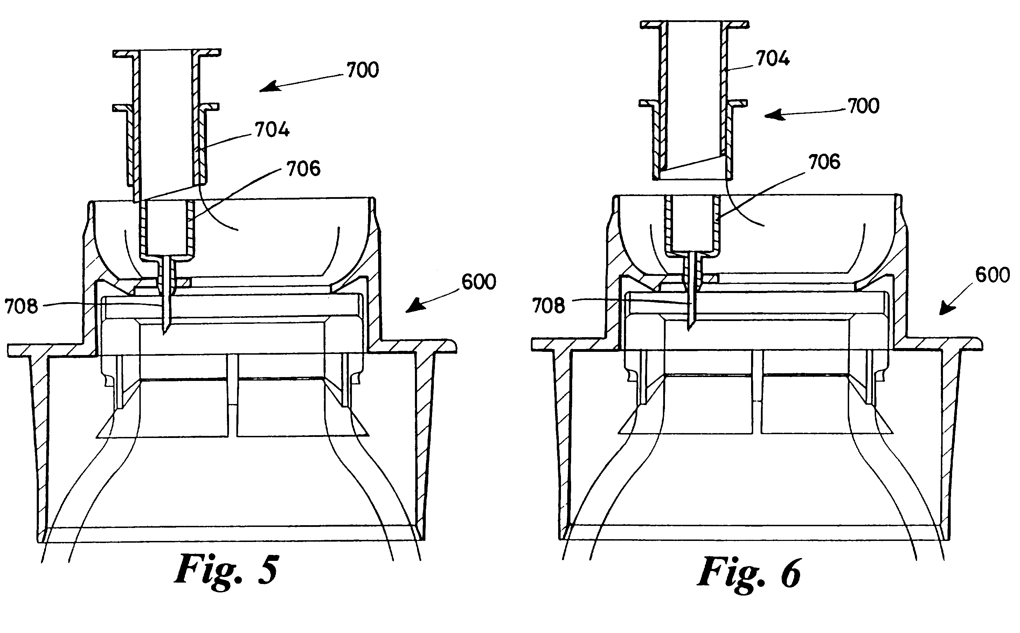

In operation, first the guide tube 704 is advanced through the outer sleeve so that its forward end cuts and deflects the foil 712 out of the path of the needle (Figure 2). The needle carrier 706 at the time advances with the guide tube 704. The advance of the guide tube 704 is then continued, downwardly into the throat 622 of the overcap 600 of the culture bottle (Figure 3). Advance of the guide tube 704 then ceases, and instead the needle carrier 706 is advanced through the guide tube. The forward end portion 710 of the needle carrier 706 enters the aperture in the guide tab 626 at the bottom of the overcap throat 622, ensuring correct registration of the venting needle 708, which pierces the septum of the closure 614 of the culture bottle (Figure 4). Excess pressure in the bottle is thus safely relieved.

The guide tube 704 is then retracted upwardly (Figure 5) back to its starting position (Figure 6), leaving the needle carrier 706 in position in the throat 622 of the overcap 600, with the needle 708 penetrating the bottle septum. The needle carrier 706 is accommodated within the depth of the overcap throat 622. The spent outer sleeve and guide tube assembly is then disposed of. The needle carrier and needle are disposed of with the culture bottle after testing and examination have been completed.

The most important point to note about the venting device is that sterility of the needle 708 is maintained substantially up to the moment of penetration of the bottle closure septum. In particular, the use of the guide tube 704 to cut and deflect the foil 712 ensures that the needle does not contact this foil during its downward movement.

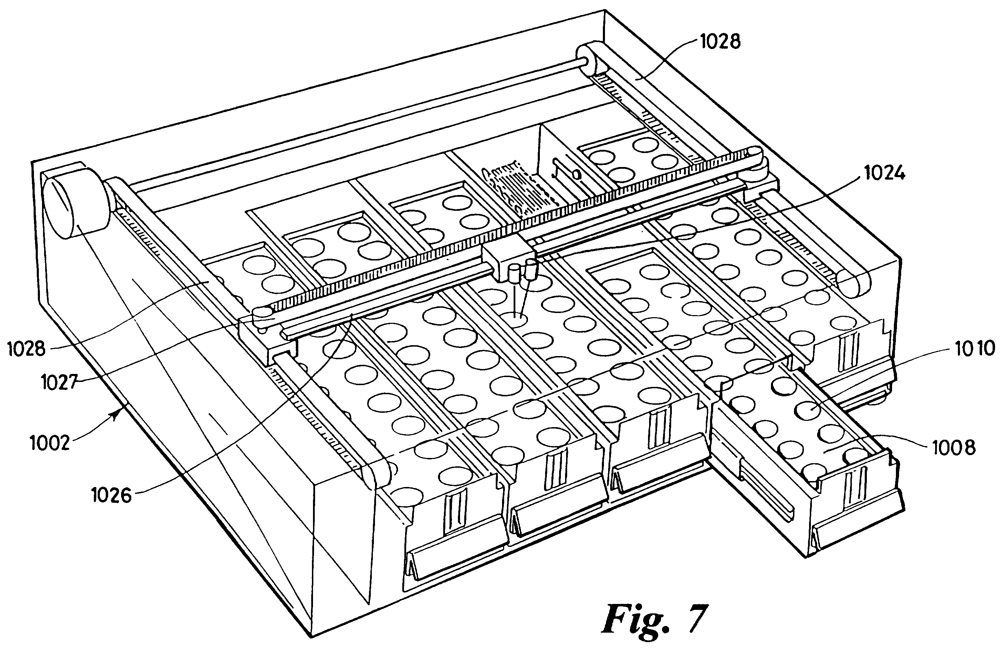

Figure 7 shows an embodiment of culture bottle monitoring apparatus comprising a stationary flat bed in which a plurality of bottles are located, and a movable laser arranged repeatedly to scan the bottles in turn.

The flat bed 1002, which may comprise one drawer of an incubation cabinet, comprises five similar supports 1008, each slidably received therein and independently removable therefrom. Each support 1008 comprises two apertured aluminium blocks defining two side by side rows of ten similar recesses 1010, each for receiving a respective culture bottle (not shown).

The bed 1002 further comprises a laser 1024 (e.g. a Matsushita LA40 laser) mounted for movement in two perpendicular directions (X and Y) on a generally conventional X - Y motion controller. The controller comprises a cross-rail 1026 on which laser 1024 is mounted for sliding movement in an X direction under the action of a stepper-motor driven belt drive 1027. The ends of rail 1024 are carried by stepper-motor driven belt drives 1028, for causing movement in a Y direction.

In use, sample containing bottles are loaded into recesses 1010. In normal use, the laser performs a scanning cycle once every five minutes. Scanning a full bed takes about two and a half minutes. This is followed by a rest period of two and a half minutes for a full bed. The results of each scan of each bottle are conveyed to a computer control means for suitable processing and display on a VDU if appropriate. When a positive result is detected for a particular bottle, this information is made available to an operator, e.g. by a suitable message on the VDU.

Further and more complete details of the monitoring apparatus of Figure 7 can be found in Patent Application No. PCT/GB92/01327 (WO93/03178).

If an indication is given on the VDU that an excess pressure has developed in one of the culture bottles, possibly also identified at the LED associated with each bottle overcap, it is appropriate for safety reasons to use the venting device of the present invention to vent the bottle in question and relieve the excess pressure.

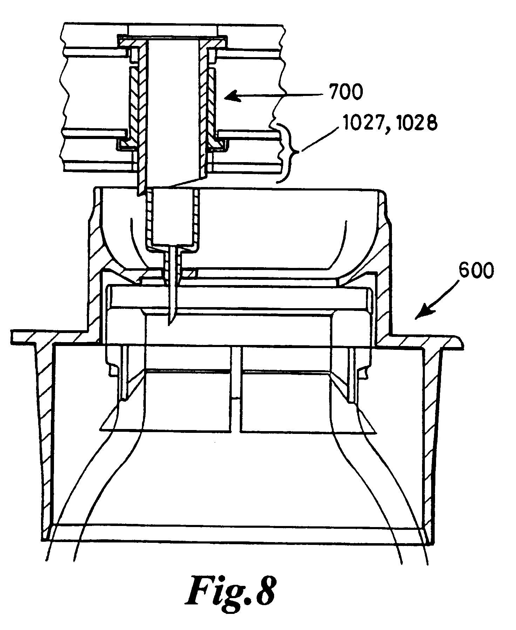

Conveniently, as indicated in Figure 8, the drive mechanism 1026, 1027, 1028 of the scanner 1024 may be used to move a venting device to the particular bottle to be vented. In practice, the venting device, possibly together with a mechanism for effecting automatic operation thereof, can be carried by the laser 1024, and utilised during the two and a half minute rest period of the laser. This is a practicable procedure because only a very small proportion of the culture bottles in the bed will require venting. Thus, the venting mechanism may carry a small stack, say five, disposable venting devices, and the computer may monitor the stack level and call for replacements as necessary.

In addition to a magazine or carrier for replacing a spent venting device, the venting mechanism includes a drive for operating the venting device. One possible drive is a lead screw which firstly acts on the guide tube to move it through the outer sleeve (which is held fixed on the laser) and secondly acts on a plunger to move the needle carrier through the guide tube after the latter has been engaged against a stop or a limit switch has operated. In a modification, a passive drive for the needle carrier is used, wherein the second function of the lead screw is to lower a loading weight on to the needle carrier. The lead screw is a reversible drive to lift the outer sleeve and guide tube, and if used the loading weight, back to the start position after the needle has pierced the bottle closure septum. All the motorised actions are preferably controlled by limit switches. Additionally, the complete operating sequence is microchip controlled under the supervision of the computer.

## Claims

1. A venting device for a culture bottle having a septum, comprising a needle carrier (706) having a venting volume in communication with a needle (708), and a membrane-carrying structure (702) within which the needle carrier and needle are movably sealed, the arrangement being such that the device is operable, when positioned with the membrane (712) between the needle (708) and the septum, firstly to move the membrane away from its position between the needle and the septum and secondly to move the needle to pierce the septum in order to vent excess volume into the venting volume.

2. A venting device according to claim 1, wherein the membrane-carrying structure comprises an outer sleeve (702) and the needle carrier (706) is slidable through a guide tube (704) slidable through the outer sleeve (702) to move the membrane (712) and to enable movement of the needle carrier (706) through the guide tube (704) to pierce the septum.

3. A venting device according to claim 2, wherein the outer sleeve (702) is closed at one end by a frangible membrane (712), and the device has an unused rest condition in which the forward end of the guide tube (704) is retracted behind the membrane (712) and the forward end of the needle (708) is retracted behind the forward end of the guide tube (704), an intermediate operational condition in which the guide tube has been moved forwardly to cut and deflect the membrane (712) out of the path of the needle, and a final operational condition in which the needle carrier (706) has been moved forwardly through the guide tube (704), in use to enable the needle (708) to penetrate the septum of the culture bottle and vent the bottle to the interior of the needle carrier behind the needle.

4. A venting device as claimed in claim 3, wherein the interior of the needle carrier (706) behind the needle contains a microbiologically effective filter for assisting the prevention of movements of organisms into or out of the bottle.

5. A venting device as claimed in claim 3 or 4, wherein the needle carrier is releasable out of the forward end of the guide tube, whereby in use the needle remains in place extending through the septum of the culture bottle.

6. A venting device as claimed in claim 5, in the form of a sterile package, the outer sleeve and guide tube being disposable after use and the needle carrier and needle being disposable with the culture bottle when microbiological testing has been completed.

7. A venting device as claimed in claim 5 or 6, in combination with an overcap (600) for the culture bottle which has an entry throat (622) in which the needle carrier is accommodated when the needle is left extending through the septum of the culture bottle.

8. The combination claimed in claim 7, wherein the entry throat of the overcap carries an axial guide (626) for a part of the needle carrier of reduced cross section in which the needle is mounted.

9. Culture bottle examination apparatus which includes means for detecting pressure variations in the bottle, a venting device in accordance with any of claims 1 to 8, and means for operating the venting device if an excess bottle pressure is detected.

10. Examination apparatus as claimed in claim 9, comprising a multibottle carrier (1002,1009) for a plurality of culture bottles, means (1024) for repeatedly scanning the bottles to detect pressure variations in the bottles, and means (1026,1027,1028) for moving a venting device into an operating position at any bottle at which an excess pressure is detected.

11. Examination apparatus as claimed in claim 10, in which the scanning mechanism for a pressure detecting means is also employed to move the venting device to a bottle requiring venting.

## Patentansprüche

1. Entlüftungsvorrichtung für eine Kulturflasche mit einer Scheidewand, welche einen Nadelträger (706) mit einem mit einer Nadel (708) in Verbindung stehenden Entlüftungsvolumen sowie eine membrantragende Struktur (702) aufweist, innerhalb der der Nadelträger und die Nadel beweglich abgedichtet sind, wobei die Anordnung derart gestaltet ist, daß die Vorrichtung in der Position mit der Membran (712) zwischen der Nadel (708) und der Scheidewand erstens zum Bewegen der Membran weg von ihrer Position zwischen der Nadel und der Scheidewand und zweitens zum Bewegen der Nadel zum Durchstechen der Scheidewand, um das übermäßige Volumen in das Entlüftungsvolumen zu entlüften, betätigbar ist.

2. Entlüftungsvorrichtung nach Anspruch 1, wobei die membrantragende Struktur eine äußere Buchse (702) aufweist und der Nadelträger (706) durch eine Führungsröhre (704) durch die äußere Buchse (702) verschiebbar ist, um die Membran (712) zu bewegen und eine Bewegung des Nadelträgers (706) durch die Führungsröhre (704) zum Durchstechen der Scheidewand zu ermöglichen.

3. Entlüftungsvorrichtung nach Anspruch 2, wobei die äußere Buchse (702) an einem Ende durch eine brechbare Membran (712) geschlossen ist und die Vorrichtung eine Nichtbenutzungs-Ruhestellung, in der das Vorderende der Führungsröhre (704) hinter die Membran (712) zurückgezogen ist und das Vorderende der Nadel (708) hinter das Vorderende der Führungsröhre (704) zurückgezogen ist, eine mittlere Betriebsstellung, in der die Führungsröhre nach vorne zum Durchschneiden und Ablenken der Membran (712) aus dem Weg der Nadel bewegt ist, sowie eine Endbetriebsstellung aufweist, in der der Nadelträger (706) nach vorne durch die Führungsröhre (704) bewegt ist, um bei der Benutzung zu ermöglichen, daß die Nadel (708) die Scheidewand der Kulturflasche durchdringt und die Flasche in das Innere des Nadelträgers hinter der Nadel entlüftet.

4. Entlüftungsvorrichtung nach Anspruch 3, wobei das Innere des Nadelträgers (706) hinter der Nadel einen mikrobiologisch wirksamen Filter enthält, um die Verhinderung von Bewegungen von Organismen in die oder aus der Flasche zu unterstützen.

5. Entlüftungsvorrichtung nach Anspruch 3 oder 4, wobei der Nadelträger aus dem Vorderende der Führungsrohre entfernbar ist, wodurch die Nadel bei der Benutzung an ihrem Ort durch die Scheidewand der Kulturflasche verlaufend bleibt.

6. Entlüftungsvorrichtung nach Anspruch 5 in Form einer sterilen Packung, wobei die äußere Buchse und die Führungsröhre nach der Benutzung wegwerfbar sind und der Nadelträger und die Nadel mit der Kulturflasche nach Beendigung des mikrobiologischen Tests wegwerfbar sind.

7. Entlüftungsvorrichtung nach Anspruch 5 oder 6 in Kombination mit einer Überzugverschlußkappe (600) für die Kulturflasche, die einen Eintrittshals (622) aufweist, in dem der Nadelträger enthalten ist, wenn die Nadel durch die Scheidewand der Kulturflasche verlaufend zurückgelassen ist.

8. Kombination nach Anspruch 7, wobei der Eintrittshals der Überzugverschlußkappe eine axiale Führung (626) für einen Teil des Nadelträgers mit reduziertem Querschnitt aufweist, in dem die Nadel angebracht ist.

9. Kulturflaschen-Untersuchungsvorrichtung mit einer Einrichtung zum Erfassen von Druckänderungen in der Flasche, einer Entlüftungsvorrichtung nach einem der Ansprüche 1 bis 8 sowie einer Einrichtung zum Betreiben der Entlüftungsvorrichtung, falls ein übermäßiger Flaschendruck erfaßt ist.

10. Untersuchungsvorrichtung nach Anspruch 9, aufweisend einen Mehrflaschenträger (1002, 1009) für eine Vielzahl von Kulturflaschen, eine Einrichtung (1024) zum wiederholten Scannen der Flaschen zur Erfassung von Druckänderungen in den Flaschen sowie eine Einrichtung (1026, 1027, 1028) zum Befördern einer Entlüftungsvorrichtung in eine Betriebsposition an irgendeiner Flasche, an der ein übermäßiger Druck erfaßt ist.

11. Untersuchungsvorrichtung nach Anspruch 10, wobei der Scanmechanismus für eine Druckerfassungseinrichtung auch zur Beförderung der Entlüftungsvorrichtung zu einer Flasche, welche eine Entlüftung benötigt, verwendbar ist.

## Revendications

1. Dispositif de décompression pour flacon de culture ayant un septum, comportant un support d'aiguille (706) ayant un volume de décompression en communication avec une aiguille (708), et une structure supportant une membrane (702) dans laquelle le support d'aiguille et l'aiguille sont agencés de manière mobile et étanche, l'agencement étant tel que le dispositif peut être actionné, lorsqu'il est positionné avec la membrane (712) située entre l'aiguille (708) et le septum, tout d'abord pour déplacer la membrane en l'éloignant de sa position située entre l'aiguille et le septum et deuxièmement pour déplacer l'aiguille pour percer le septum afin de décompresser le volume en excès à l'intérieur du volume de décompression.

2. Dispositif de décompression selon la revendication 1, dans lequel la structure supportant une membrane comporte un manchon extérieur (702) et le support d'aiguille (706) peut coulisser le long d'un tube de guidage (704) pouvant coulisser le long du manchon extérieur (702) pour déplacer la membrane (712) et permettre le mouvement du support d'aiguille (706) le long du tube de guidage (704) pour percer le septum.

3. Dispositif de décompression selon la revendication 2, dans lequel le manchon extérieur (702) est fermé au niveau d'une première extrémité par une membrane fragile (712), et le dispositif a un état de repos non-utilisé dans lequel l'extrémité avant du tube de guidage (704) est rétractée derrière la membrane (712) et l'extrémité avant de l'aiguille (708) est rétractée derrière l'extrémité avant du tube de guidage (704), un état opérationnel intermédiaire dans lequel le tube de guidage a été déplacé vers l'avant pour découper et infléchir la membrane (712) en dehors du trajet de l'aiguille, et un état de fonctionnement final dans lequel le support d'aiguille (706) a été déplacé vers l'avant le long du tube de guidage (704), en utilisation pour permettre à l'aiguille (708) de pénétrer dans le septum du flacon de culture et de décompresser le flacon à l'intérieur du support d'aiguille derrière l'aiguille.

4. Dispositif de décompression selon la revendication 3, dans lequel l'intérieur du support d'aiguille (706) situé derrière l'aiguille comporte un filtre micro-biologiquement efficace pour aider à l'empêchement des mouvements d'organismes vers l'intérieur ou vers l'extérieur du flacon.

5. Dispositif de décompression selon l'une quelconque des revendications 3 ou 4, dans lequel le support d'aiguille peut être libéré de l'extrémité avant du tube de guidage, de sorte qu'en utilisation l'aiguille reste en place en s'étendant à travers le septum du flacon de culture.

6. Dispositif de décompression selon la revendication 5, ayant la forme d'un emballage stérile, le tube de guidage et le manchon extérieur pouvant être jetés après utilisation et le support d'aiguille et l'aiguille pouvant être jetés avec le flacon de culture lorsque les tests microbiologiques ont été réalisés.

7. Dispositif de décompression selon la revendication 5 ou 6, en combinaison avec un couvercle de recouvrement (600) pour le flacon de culture qui a une gorge d'entrée (622) dans laquelle est agencé le support d'aiguille lorsque l'aiguille est laissée s'étendant à travers le septum du flacon de culture.

8. Combinaison selon la revendication 7, dans laquelle la gorge d'entrée du couvercle de recouvrement supporte un guide axial (626) pour une partie du support d'aiguille ayant une section transversale réduite dans laquelle est agencée l'aiguille.

9. Dispositif d'examen de flacon de culture qui comporte des moyens pour détecter des variations de pression dans le flacon, un dispositif de décompression selon l'une quelconque des revendications 1 à 8, et des moyens pour actionner le dispositif de décompression si une pression de flacon excessive est détectée.

10. Dispositif d'examen selon la revendication 9, comportant un support de plusieurs flacons (1002, 1005) pour plusieurs flacons de culture, des moyens (1024) pour balayer d'une manière répétée les flacons pour détecter les variations de pression dans les flacons, et des moyens (1026, 1027, 1028) pour déplacer un dispositif de décompression dans une position active au niveau de tout flacon dans lequel est détectée une pression excessive.

11. Dispositif d'examen selon la revendication 10, dans lequel le mécanisme de balayage des moyens de détection de pression est aussi utilisé pour déplacer le dispositif de décompression vers un flacon nécessitant une décompression.
